# EUROPEAN PATENT APPLICATION

(11) **EP 4 094 788 A1**
(43) Date of publication of application: **30.11.2022**
(21) Application number: 20915061.4
(22) Date of filing: 24.07.2020
(51) Int. Cl.: A61M 5/19, A61M 5/14, A61M 5/168, A61M 5/178

(54) **GAS AND DRUG SEQUENTIAL INJECTION MODULE FOR SKIN TREATMENT, AND SKIN TREATMENT DEVICE COMPRISING SAME**

(30) Priority: 23.01.2020 KR 20200009301
(71) Applicant: Mcure Co. Ltd., Gangwon-do 26311 (KR)
(72) Inventor: LEE, Man Kyu, Seongnam-si Gyeonggi-do 13525 (KR); SON, Ho Sung, Wonju-si Gangwon-do 26473 (KR); CHOI, Byung Wook, Wonju-si Gangwon-do 26311 (KR); AN, Ick Jong, Wonju-si Gangwon-do 26352 (KR)
(74) Representative: Vossius & Partner Patentanwälte Rechtsanwälte mbB
(86) International application number: PCT/KR2020/009819
(87) International publication number: WO 2021/149885

(57) **Abstract**

Provided is a sequential injection module included in a skin treatment device, the module sequentially injecting a gas and a drug into the skin of a user through one needle. The sequential injection module comprises: a supply port for supplying each of gas and a drug; an output port for discharging each of same; and a channel control part for forming, therein, either a gas flow channel or a drug flow channel by means of the pressure of the gas and drug to be supplied.

## Description

### [Technical Field]

The present disclosure relates to a gas and drug sequential injection module for skin treatment, the module mounted on a skin treatment device and sequentially injecting a gas and drug into the skin of a patient, and a skin treatment device including the same.

### [Background Art]

Mesotherapy, which performs a medical procedure by injecting a gas or air and a drug into a skin is a kind of injection treatments that is performed for diseases in the fields of acute/chronic pain, partial corpulence, cosmetic surgery, etc., and is a medical procedure of injecting a small amount of treatment drug into a skin layer and a subcutaneous layer using specific injection instrument and needle.

Mesotherapy has the advantage that it is possible to minimize general oversensitive reaction and side effects due to drugs and treat only desired portions by injecting a small amount of drug into subcutaneous tissues to be treated. Recently, mesotherapy is generally used for cosmetic treatment and disease treatment such as corpulence treatment, pain relief treatment, skin elasticity improvement, and scar treatment.

A method using a mezo gun and a manual method are common procedures of mesotherapy, and it is general to use a needle connected to a tube for supplying a drug and gas/air.

For example, an operator puts a needle into a skin layer and injects gas or air, and a drug into the skin layer through the needle so that wrinkles of the skin can be removed by the treatment drug injected in the skin layer, whereby a medical procedure is performed.

An instrument that can perform a medical procedure by sequentially injecting a gas and a drug into a skin, and a catheter for the instrument have been disclosed in Korean Patent Application Publication No. 10-2012-0039870.

Such a skin treatment instrument is a device that can treat a skin by first injecting air or gas into a treatment site of a skin before injecting a drug into the treatment site and then injecting a drug after a space is secured by separating a skin layer and a subcutaneous layer by lifting the caved skin layer with the injected air or gas, and uses a catheter having two needles for injecting air or gas and a drug, respectively.

That is, such a skin treatment instrument has separate needles for injecting a drug and gas or air, respectively, and performs a medical procedure by injecting a drug and gas or air with the two needles put in a skin.

However, since the skin treatment instrument in the related art uses two needles for a medical procedure, there is a problem that a patient feels a severe pain or scars remain.

Further, since the needles for supplying gas or air and a drug into a catheter, respectively, are spaced apart from each other in the skin treatment instrument in the related art, the sites to which gas is injected and a drug is injected are different in a skin, so efficiency of the medical procedure is deteriorated.

### [Summary of Invention]

### [Technical Problem]

The present disclosure has been made in an effort to solve the problems described above, and an objective of the present disclosure is to provide a gas and drug sequential injection module for skin treatment, the module sequentially injecting a gas and a drug into a skin of a patient through one needle, and a skin treatment device including the module.

### [Solution to Problem]

A sequential injection module according to an embodiment of the present disclosure is provided for a skin treatment device that sequentially injects a gas and a drug into the skin of a user through one needle.

The sequential injection module includes: a gas input port being supplied with the gas into a first cavity therein; a drug input port being supplied with the drug into a second cavity therein; an output port discharging one of the gas and the drug to the needle through a third cavity therein; and a channel controller forming one of a gas flow path and a drug flow path while being moved horizontally by each of the gas and the drug between the first cavity, the second cavity, and the third cavity.

Further, a skin treatment device having the sequential injection module according to an embodiment of the present disclosure includes: a body including a discharge controller controlling discharge of a drug and a gas respectively from a drug container and a gas container accommodated therein; and a sequential injection module coupled between an end of the body and a holder to which the needle is coupled, and providing the drug and the gas supplied from the body to the needle.

### [Advantageous Effects of Invention]

The skin treatment device having a gas and drug sequential injection module for skin treatment of the present disclosure can provide a medical procedure for skin improvement such as removal of wrinkles without a burden such as a pain of a patient by sequentially injecting a gas and a drug into the skin acupunctured by the sequential injection module with one needle inserted in the skin of the patient.

Further, since the skin treatment device of the present disclosure injects a gas and a drug to the same point in the skin of a patient through one needle, it is possible to increase the effect of a medical procedure by a gas and a drug for skin improvement of a patient.

### [Brief Description of Drawings]

FIG. 1 is a view showing a skin treatment device equipped with a sequential injection module according to an embodiment of the present disclosure.
FIG. 2 is an enlarged view of the part A of FIG. 1.
FIG. 3 is an exploded perspective view of the sequential injection module according to an embodiment of the present disclosure.
FIGS. 4A and 4b are cross-sectional views for describing operation of the sequential injection module shown in FIG. 3.

### [Description of Embodiments]

Hereafter, the configuration and operation of embodiments of the present disclosure are described with reference to the accompanying drawings.

It should be noted that even though same components are shown in different drawings, they are given the same reference numerals and characters. In the following description of the present disclosure, detailed descriptions of well-known functions or configurations relating to the disclosure will not be provided so as not to obscure the description of the disclosure with unnecessary details. Further, unless explicitly described otherwise, 'comprising' any components will be understood to imply the inclusion of other components rather than the exclusion of any other components.

The terms and words used in the present specification and claims should not be interpreted as being limited to typical meanings or dictionary definitions, but should be interpreted as having meanings and concepts relevant to the technical scope of the present disclosure based on the rule according to which the inventors can appropriately define the concept of the term to describe most appropriately the best method he or she knows for carrying out the disclosure. Accordingly, the embodiments described herein and the configurations shown in the drawings are only preferred embodiments of the present disclosure and do not represent all of the spirits of the present disclosure. Therefore, there may be equivalents and modifications that can replace them, and the scope of the present disclosure is not limited to the embodiments to be described hereafter.

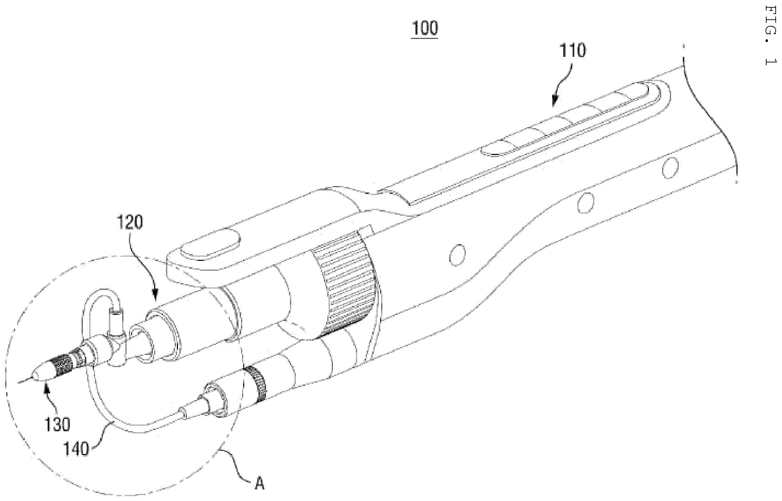

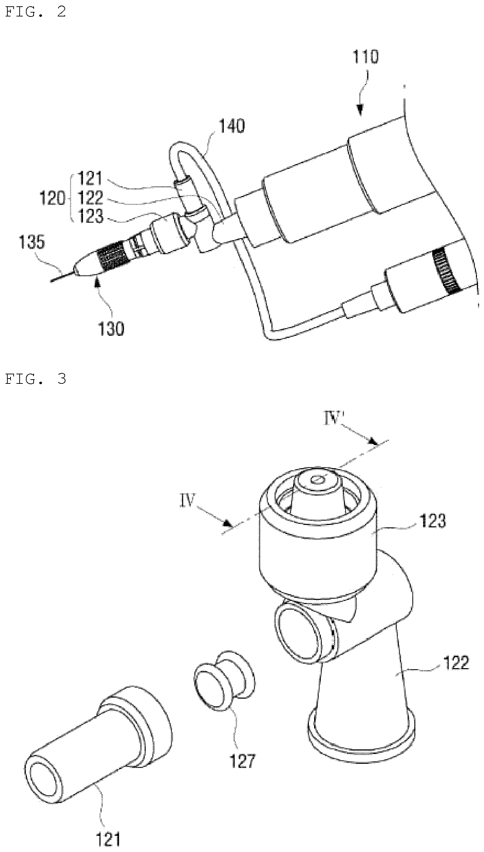

FIG. 1 is a view showing a skin treatment device equipped with a sequential injection module according to an embodiment of the present disclosure, and FIG. 2 is an enlarged view of the part A of FIG. 1.

Referring to FIGS. 1 and 2, a skin treatment device 100 according to the present disclosure may include a body 110, a sequential injection module 120, and a holder 130. Further, a needle 135 having a predetermined length and configured to be put into the skin of a user, that is, a patient, may be coupled to the holder 130.

The skin treatment device 100 can inject a gas and a drug into the skin of a user through the needle 145 by sequentially supplying the gas and the drug into the holder 130 through the sequential injection module 120 from the body 110 in a state where the needle 135 is put in the skin of the user. Accordingly, a user can have a skin treatment for skin improvement such as removal of wrinkles by a gas and a drug that are injected from the skin treatment device 100.

The gas may be nitrogen, oxygen, or carbon dioxide. Further, a gas and a drug can be sequentially injected into the skin of a user by operation of the sequential injection module 120.

A drug container (not shown) keeping a drug and a gas container (not shown) keeping a gas may be accommodated in the body 110. Further, the body 110 may have a discharge controller (not shown) that separately controls discharge of a drug and a gas.

The body 110 can discharge a drug through a drug outlet (not shown) from the drug container in a predetermined amount under a predetermined pressure in accordance with operation of the discharge controller. Further, the body 110 can discharge a gas through a gas outlet (not shown) from the gas container in a predetermined amount under a predetermined pressure in accordance with operation of the discharge controller.

The discharge controller can control the body 110 such that a drug and a gas are sequentially discharged from the body 110. Further, the discharge controller can separately control the discharge amounts and the discharge pressure of a drug and a gas in accordance with a preset pressure.

The sequential injection module 120 may be disposed between the body 110 and the holder 130 and coupled thereto, respectively. The sequential injection module 120 can supply the drug and gas, which are supplied from the body 110, to the holder 130.

The sequential injection module 120 may have a gas input port 121 coupled to the gas outlet of the body 110, a drug input port 122 coupled to the drug outlet of the body 110, and an output port 123 coupled to the holder 130. The gas input port 121 may be connected to the gas outlet through a supply tube 140.

All or at least two of the gas input port 121, drug input port 122, and output port 123 of the sequential injection module 120 may be integrated. Further, a cavity may be formed in each of the ports, that is, each of the gas input port 121, drug input port 122, and output port 123 such that a drug and a gas can flow in the sequential injection module 120.

Further, a channel controller (127 in FIG. 3) that controls components for the channel of a drug and the flow path of a gas may be disposed in the sequential injection module 120. A gas supplied to the gas input port 121 can be provided to the holder 130 through the output port 123 or a drug supplied to the drug input port 122 can be provided to the holder 130 through the output port 123 by the channel controller 127.

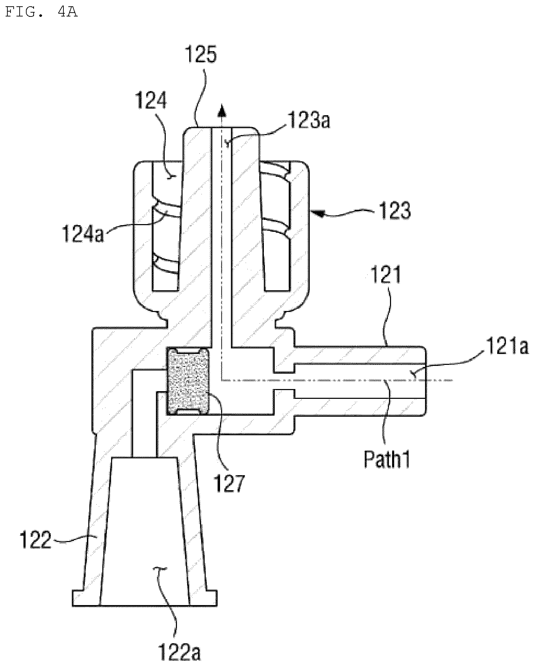

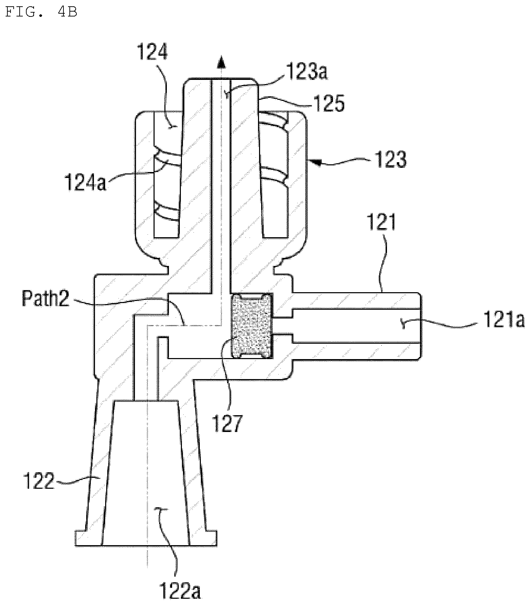

FIG. 3 is an exploded perspective view of the sequential injection module according to an embodiment of the present disclosure, and FIGS. 4A and 4B are cross-sectional views for describing operation of the sequential injection module shown in FIG. 3.

Referring to the drawings, the sequential injection module 120 of the present disclosure has a gas input port 121, a drug input port 122, and an output port 123 at least two of which are integrated, and a channel controller 127 may be disposed in the ports.

Cavities, that is, a first cavity 121a, a second cavity 122a, and a third cavity 123a may be formed in the gas input port 121, drug input port 122, and output port 123, respectively. The first cavity 121a and the third cavity 123a may communicate with each other in the sequential injection module 120.

The gas input port 121 may be connected to the gas outlet of the body 110. The supply tube 140 connected with the gas outlet of the body 110 may be inserted and coupled in the first cavity 121a of the gas input port 121.

The drug input port 122 may be connected to the drug outlet of the body 110. The drug outlet of the body 110 may be inserted and coupled in the second cavity 122a of the drug input port 122.

The output port 123 may be connected to the holder 130. The holder 130 may be detachably coupled to the output port 123. To this end, a coupling portion 124 and protrusion 125 may be formed at the output port 123.

A spiral groove 124a may be formed on the inner surface of the coupling portion 124 of the output port 123. Accordingly, it is possible to couple or separate the holder 130 to or from the output port 123 along the spiral groove 124a by inserting the holder 130 into the coupling portion 124 and then turning the holder 130 in a predetermined direction. Of course, a spiral protrusion (not shown) corresponding to the spiral groove 124a may be formed on the outer surface of the holder 130.

Accordingly, it is possible to separate and remove only the holder 130 from the sequential injection module 120 of the skin treatment device 100 after injecting a gas and a drug into the skin of a user.

The protrusion 125 of the output port 123 can be inserted into the holder 130 coupled to the coupling portion 124. The third cavity 123a may be formed in the protrusion 125. The third cavity 123a can supply one of a gas and a drug that are supplied from one of the first cavity 121a of the gas input port 121 and the second cavity 122a of the drug input port 122 to the holder 130.

The channel controller 127 may be disposed in a fourth cavity 127a formed in the sequential injection module 120, that is, in a fourth cavity 127a formed between the first cavity 121a and the third cavity 123a. The channel controller 127 can be moved left and right, that is, horizontally in the fourth cavity 127a by pressure of the gas that is supplied through the first cavity 121a or the pressure of the drug that is supplied through the second cavity 122a.

The channel controller 127a may be made of silicone, etc. Further, the channel controller 127 may have an H-shape to easily move in the fourth cavity 127a. Accordingly, the channel controller 127 is partially in contact with the inner surface, that is, the top and the bottom of the fourth cavity 127a, whereby friction can be reduced when the channel controller 127 is horizontally moved.

As the channel controller 127 moves, a first path Path 1 through which a gas flows and a second path Path 2 through which a drug flows may be formed in the sequential injection module 120. That is, the channel controller 127 is horizontally moved by external pressure, thereby forming paths for a gas or a dug to be sequentially injected into the skin of a user through the sequential injection module 120 and the holder from the body 110.

Referring to FIG. 4A, when a gas at a predetermined pressure is discharged from the body 110 and supplied to the gas input port 121 of the sequential injection module 120, the channel controller 127 of the sequential injection module 120 can be moved horizontally in a first direction, that is, toward the second cavity 122a by the pressure of the gas flowing inside through the first cavity 121a of the gas input port 121.

The outlet of the second cavity 122a is closed by this movement of the channel controller 127, whereby the first path Path 1 that communicates with the first cavity 121a, the fourth cavity 124a, and the third cavity 123a can be formed. Further, a gas that is supplied from the body 110 can be provided to the holder 130 along the first path Path 1 of the sequential injection module 120 and then provided to the needle 135 coupled to the holder 130, whereby the gas can be injected into the skin of a user.

In this process, the needle may have been put in between a skin layer and a subcutaneous layer of the skin of the user. Accordingly, the gas supplied from the sequential injection module 120 can form a space between the skin layer and the subcutaneous layer of the skin of the user through the needle 135.

Referring to FIG. 4B, when a drug at a predetermined pressure is discharged from the body 110 and supplied to the drug input port 122 of the sequential injection module 122, the channel controller 127 of the sequential injection module 120 can be moved horizontally in a second direction, that is, toward the first cavity 121a by the pressure of the drug flowing inside through the second cavity 122a of the drug input port 122.

The outlet of the first cavity 121a is closed by this movement of the channel controller 127, whereby the gas that is being supplied is blocked and the second path Path 2 that communicates with the second cavity 122a, the fourth cavity 124a, and the third cavity 123a can be formed. Further, a drug that is supplied from the body 110 can be provided to the holder 130 along the second path Path 2 of the sequential injection module 120 and then provided to the needle 135 coupled to the holder 130, whereby the gas can be injected into the skin of a user.

In this process, a space may have been formed between the skin layer and the subcutaneous layer of the skin of the user by the needle inserted in the skin and the supplied gas. Accordingly, the drug supplied from the sequential injection module 120 can be injected into the space formed in the skin of the user through the needle 135. Therefore, a medical procedure for skin improvement of the user such as removal of wrinkles can be achieved as an optimal amount of drug is effectively injected into between the skin layer and the subcutaneous layer of the skin of the user.

As described above, according to the sequential injection module 120 of the present embodiment, the respective pressures of a gas and a drug supplied from the body 110 can move the channel controller therein horizontally, thereby forming a gas flow path and a drug flow path, respectively.

Accordingly, the skin treatment device 100 of the present disclosure can provide a medical procedure for skin improvement such as removal of wrinkles without a burden such as a pain of a user by sequentially injecting a gas and a drug into the skin acupunctured by the sequential injection module 120 with one needle 135 inserted in the skin of the user.

Further, since the skin treatment device 100 of the present disclosure injects a gas and a drug to the same point in the skin of a user through one needle 135, it is possible to increase the effect of a medical procedure for skin improvement of a user.

Meanwhile, as described with reference to FIG. 4B, after the second path Path 2 is formed by the channel controller 127 of the sequential injection module 120 and injection of a drug into a space in the skin of a user is completed, that, a space formed by the previously injected gas, the needle 135 inserted in the skin can be pulled out.

In this case, since a predetermined amount of drug remains in the second cavity 122a, the third cavity 123a, and the fourth cavity 127a, the drug flows out through the tip of the needle 135 due to a pressure change by pulling-out of the needle 135.

In order to prevent this problem, the skin treatment device 100 may perform suck back for decreasing pressure inside the second cavity 122a after injecting a drug through the second path Path 2 of the sequential injection module 120. Accordingly, even though the needle 135 of the skin treatment device 100 is pulled out of the skin of a user, the drug remaining in the sequential injection module 120 does not flow outside due to a pressure drop inside the second cavity 122a.

For example, a piston (not shown) may be coupled in the drug container accommodated in the body 110 of the skin treatment device 100. Accordingly, the discharge controller of the body 110 can pressurize the inside of the drug container by moving forward the piston such that the drug in the drug container is supplied to the sequential injection module 120.

Accordingly, after a drug finishes being injected into the skin of a user, the discharge controller of the body 110 can perform suck back that moves backward by a predetermined amount the piston pressurizing the inside of the drug container.

The pressure inside the sequential injection module 120, that is, the pressure of the second cavity 122a is changed and decreased by backward movement of the piston, whereby the drug remaining in the sequential injection module 120 can be prevented from flowing to the tip of the needle 135.

Meanwhile, when the skin treatment device 100 performs suck back, the channel controller 127 of the sequential injection module 120 can maintain the state where the outlet of the first cavity 121a is closed, that is, can maintain the state where inflow of a gas is blocked.

Accordingly, the channel controller 127 may be given predetermined hardness, for example, 40-60 HA, preferably 50-55 HA. Therefore, it is possible to prevent the channel controller 127 from moving from the first cavity 121a to the second cavity 122a when the pressure of the second cavity 122a is decreased by suck back. Further, it is possible to prevent leakage of a gas or a drug due to wear by frequent movement of the channel controller 127 in the fourth cavity 127a.

As described above, the skin treatment device 100 can sequentially supply a gas and a drug to the needle 135 from the body 110 through the sequential injection module 120 coupled between the body 110 and the needle 135.

Accordingly, the skin treatment device 100 of the present disclosure can provide a skin improvement effect such as removal of wrinkles without a burden such as a pain of a user by sequentially injecting a gas and a drug into the portion acupunctured by the sequential injection module 120 with one needle 135 inserted in the skin of the user. Further, since the skin treatment device 100 of the present disclosure injects a gas and a drug to the same point in the skin of a user through one needle 135, it is possible to increase the effect of a medical procedure for skin improvement of a user.

### [Industrial Applicability]

The present disclosure can be used for a medical procedure for a skin, and particularly, may be used as a skin treatment instrument that sequentially injects a gas and a drug into a skin.

## Claims

1. A sequential injection module for injecting a gas and a drug into a skin of a user through one needle, the sequential injection module comprising:
a gas input port being supplied with the gas into a first cavity therein;
a drug input port being supplied with the drug into a second cavity therein;
an output port discharging one of the gas and the drug to the needle through a third cavity therein; and
a channel controller forming one of a gas flow path and a drug flow path while being moved horizontally by each of the gas and the drug between the first cavity, the second cavity, and the third cavity.

2. The sequential injection module of claim 1, wherein the channel controller forms the gas flow path communicating from the first cavity to the third cavity by being moved toward the second cavity by pressure of the gas that is supplied to the first cavity.

3. The sequential injection module of claim 1, wherein the channel controller forms the drug flow path communicating from the second cavity to the third cavity by being moved toward the first cavity by pressure of the drug that is supplied to the second cavity.

4. The sequential injection module of claim 1, wherein the channel controller is made of silicone.

5. The sequential injection module of claim 1, wherein the channel controller is formed in an H shape.

6. The sequential injection module of claim 1, wherein the channel controller has hardness of 40-60 HA.

7. A skin treatment device for injecting a gas and a drug into a skin of a user through one needle, the skin treatment device comprising:
a body including a discharge controller controlling discharge of a drug and a gas respectively from a drug container and a gas container accommodated therein; and
a sequential injection module coupled between an end of the body and a holder to which the needle is coupled, and sequentially providing the drug and the gas supplied from the body to the needle,
wherein the sequential injection module includes:
a gas input port coupled to a gas outlet of the body and being supplied with the gas through a first cavity;
a drug input port coupled to a drug outlet of the body and being supplied with the drug through a second cavity;
an output port coupled to the holder and discharging one of the gas and the drug through a third cavity; and
a channel controller forming one of a gas flow path and a drug flow path while being moved horizontally by each of the gas and the drug between the first cavity, the second cavity, and the third cavity.

8. The skin treatment device of claim 7, wherein the channel controller forms the gas flow path communicating from the first cavity to the third cavity by being moved toward the second cavity by pressure of the gas that is supplied to the first cavity.

9. The skin treatment device of claim 7, wherein the channel controller forms the drug flow path communicating from the second cavity to the third cavity by being moved toward the first cavity by pressure of the drug that is supplied to the second cavity.

10. The skin treatment device of claim 7, wherein the channel controller is made of silicone.

11. The skin treatment device of claim 7, wherein the channel controller is formed in an H shape.

12. The skin treatment device of claim 7, wherein the channel controller has hardness of 40-60 HA.

13. The skin treatment device of claim 7, wherein the holder is detachably coupled to the output port.

14. The skin treatment device of claim 7, wherein the gas input port is coupled to the gas outlet through a gas supply tube.

15. The skin treatment device of claim 7, further comprising
a piston coupled to the drug container,
wherein the discharge controller changes pressure inside the sequential injection module by moving backward the piston after discharging the drug by moving forward the piston.
